# EUROPEAN PATENT APPLICATION

(11) **EP 2 204 170 A1**
(43) Date of publication of application: **07.07.2010**
(21) Application number: 08170384.5
(22) Date of filing: 01.12.2008
(51) Int. Cl.: A61K 9/20

(54) **Pharmaceutical composition comprising ezetimibe and simvastatin**

(71) Applicant: LEK Pharmaceuticals d.d., 1526 Ljubljana (SI)
(72) Inventor: The designation of the inventor has not yet been filed
(74) Representative: Opresnik, Marko

(57) **Abstract**

The present invention belongs to the field of pharmaceutical industry and relates to a process for preparing dosage forms containing simvastatin and ezetimibe, comprising the steps of providing a first composition containing simvastatin, providing a second composition containing ezetimibe, and forming a dosage form comprising at least two separate compartments, wherein one compartment is formed using either the first or the second composition and another compartment is formed using the other composition. The present invention also relates to a process for preparing dosage forms containing simvastatin and ezetimibe, wherein the process involves a direct compression step. Furthermore, the present invention belongs to a dosage form obtained by this process, comprising at least two separate compartments, wherein one compartment contains simvastatin and one compartment contains ezetimibe.

## Description

### Field of the invention

The present invention belongs to the field of pharmaceutical industry and relates to new dosage forms containing simvastatin and ezetimibe and a process for preparing such dosage forms.

### Description of the background art

High blood or plasma cholesterol levels or hypercholesterolemia represent a common disease pattern preliminary in the well situated countries of the western hemisphere. Cholesterol may cause a "hardening of the arteries" so that arteries become narrowed and blood flow to the heart is slowed down or even blocked with the consequence that provision of oxygen to the organs is constrained. Hypercholesterolemia has been implicated in atherosclerosis, heart attack, and stroke and is one of several conditions that may lead to coronary artery disease, which is the leading cause of death in the United States, accounting for approximately 600,000 deaths per year. The risk group includes the overweight, smokers, those with a poor diet (e.g. one rich in saturated fats), those who take inadequate exercise and suffering from stress. For such risk individuals, as well as those tested and found to have unduly high plasma cholesterol levels, a variety of treatments have been proposed, e.g. changes in diet and habits, increased exercise, etc. However, such treatments are not always easy to enforce and there exist a need for improved medicinal treatments which are effective at reducing plasma cholesterol levels.

Commonly used compounds for the treatment or prevention of high cholesterol levels in individuals are the statins, such as fluvastatin, simvastatin, and lovastatin. Among the group of statins, particularly simvastatin exhibited good results in the treatment of conditions characterized by high cholesterol levels. Methods for its preparation are disclosed e.g. in EP 0 033 538, EP 0 351 918, and EP 0 299 656. Simvastatin exerts a cholesterol reducing effect by inhibiting the conversion of 3-hydroxy-3-methylglutarylcoenzyme A (HMG-CoA) to mevalonate, an early step in the biosynthetic pathway of cholesterol. Additionally, simvastatin reduces the amount of very- low density lipoproteins (VLDL) and triglycerides (TG) and increases high-density lipoprotein cholesterol (HDL-C) and is thus capable to counteract diseases like atherosclerosis. Simvastatin is marketed worldwide and sold under the trade name ZOCOR^{®}. ZOCOR^{®} tablets contain simvastatin, anhydrous lactose, microcrystalline cellulose (fillers), pregelatinized maize starch (disintegrant), magnesium stearate (lubricant), butylated hydroxyanisol (BHA), citric acid monohydrate and ascorbic acid (antioxidants).

Also other compounds having a different mode of action with regard to a reduction of blood cholesterol levels have been proposed for use. Such a compound is for instance ezetimibe, which is described in EP 0 720 599. Ezetimibe inhibits the absorption and resorption of cholesterol, and the way of action involves increased excretions of cholesterol and its intestinally generated metabolites with the faeces. This effect results in lowered body cholesterol levels, increased cholesterol synthesis, and decreased triglyceride synthesis. The increased cholesterol synthesis initially provides for the maintenance of cholesterol levels in the circulation, levels that eventually decline as the inhibition of cholesterol absorption and resorption continues. The overall effect of drug action is the lowering of cholesterol levels in the circulation and tissues of the body. In the USA it is sold under the trade name ZETIA^{®}. Polymorphic forms of ezetimibe are for example described in WO 2005/009955.

In order to provide improved medication, combination products, such as a combination of ezetimibe and simvastatin, were considered. Such a combination product is marketed for example in the USA under the trade name VYTORIN^{®}. The commercially available VYTORIN^{®} tablets contain ezetimibe, simvastatin, lactose monohydrate, microcrystalline cellulose (fillers), hydroxylpropyl methylcellulose (binder), croscarmellose sodium (disintegrant), magnesium stearate (lubricant), butylated hydroxyanisol (BHA), citric acid monohydrate and propyl gallate (antioxidants). VYTORIN^{®} tablets are available containing 10 mg of ezetimibe each and 10, 20, 40 and 80 mg simvastatin, respectively.

Active substances such as simvastatin and ezetimibe are normally susceptible to environmental influences, such as storage temperature, humidity, light, (e.g. UV light) and gases, present in the environment, such as oxygen or carbon dioxide. An important factor is also the pH, that is, the presence of substances, which have influence on acidity or alkalinity of the environment (e.g. acids, alkalis, salts, metal oxides) and the reactivity of the ambient medium or active substance (free radicals, heavy metals), etc.. Also, excipients contained in pharmaceutical compositions may be a source of impurities and/or oxidants or metals (e.g. present impurities) and may be involved in the occurrence of mobile oxidative species, such as peroxyl-radicals, superoxide (singlet oxygen) and hydroxyl radicals. This depends on the hydrogen bond strength of the excipients and whether there are good electron donor sites (e.g. amines). Peroxide impurities are often present in polymeric excipients and they are a major cause of oxidation in pharmaceutical formulations (Waterman, K.C., et al, Stabilization of Pharmaceuticals to Oxidative Degradation, Pharmaceutical Development and Technology, 7(1), 2002, 1-32).

In order to prevent degradation and/or other undesired chemical reactions, such as oxidation reactions, stabilizers including antioxidants are normally employed.

An example of a pharmaceutical preparation comprising simvastatin, ezetimibe and antioxidants is described in US 7 229 982.

WO 2004/010993 also discloses a pharmaceutical composition comprising ezetimibe and simvastatin. The composition further includes stabilizing agents including antioxidative agents such as butylated hydroxyanisole (BHA), 2,6-di-tert-butyl-4-methyl- phenol (BHT), propyl gallate, ascorbic acid, citric acid, edeteate disodium and calcium metabisulphite.

WO 2007/003365 discloses pharmaceutical compositions wherein no stabilizing agents, particularly antioxidants, are used. The required stability of the pharmaceutical composition is reached by substantially reducing the contact of the composition with oxygen such as by coating the composition or providing the medicament in an environment having an essentially reduced oxygen content.

However, methods of substantially reducing the contact of the composition with oxygen, as described in the WO 2007/003365, require further steps and/or procedures in the preparation of pharmaceutical compositions, thereby substantially increasing the production time and/or costs.

Therefore, there is a need for an improved process for the preparation of a dosage formulation containing simvastatin and ezetimibe and for an improved dosage formulation itself.

### Summary of the invention

The present invention provides the following aspects, subject-matters and preferred embodiments, which respectively taken alone or in combination, further contribute to solving the object of the present invention:
(1) A process for preparing dosage forms comprising simvastatin and ezetimibe, the process comprising the steps of:
   a) providing a first composition containing simvastatin,
   b) providing a second composition containing ezetimibe, and
   c) forming a dosage form comprising at least two, preferably two, separate compartments,
   wherein one compartment is formed using either the first or the second composition and another compartment is formed using the respective other composition.
(2) The process according to the previous item, wherein each of the compartments respectively constitutes a layer.
(3) The process according to any of the previous two items, wherein the dosage form is a tablet, preferably a bilayer tablet, which consists of two separate compartments, each of which constitutes a layer.
   The compositions can be prepared by any available methods, such as mixing, wet granulation, dry granulation, extrusion / spheronization. Mixing is preferred.
(4) The process according to any of the previous three items, wherein steps (a) to (c) are carried out in the presence of air.
(5) The process according to any of the previous four items, wherein the first composition forms the first layer and the second composition forms the second layer,
   wherein the second layer is compressed onto the first layer.
(6) The process according to any of the previous five items, wherein to the first and to the second composition one or more excipients are respectively added, and wherein the addition of antioxidants and/or oxygen absorbers is omitted.
(7) The process according to any of the previous items, wherein excipients are added which are selected from the group consisting of diluents, binding agents, fillers, disintegrants, lubricants, sweeteners, glidants, flavourings and colouring agents.
(8) The process according to any of the previous two items, wherein the fillers are selected from the group consisting of different grades of starches, such as maize starch, potato starch, rice starch, wheat starch, pregelatinized starch, fully pregelatinized starch, cellulose, such as microcrystalline cellulose or silicified microcrystalline cellulose, mannitol, erythritol, lactose, such as lactose monohydrate and lactose anhydrous, calcium, such as calcium hydrogenphosphate, sorbitol, and xylitol, particularly preferred, the fillers are selected from the group consisting of pregelatinized starch, microcrystalline cellulose, silicified microcrystalline cellulose, lactose monohydrate, and lactose;
   the disintegrants are selected from the group consisting of carmellose calcium, carboxymethylstarch sodium, croscarmellose sodium salt (cellulose carboxymethylether sodium salt, crosslinked), starch, such as sodium starch glycolate or corn starch, crosslinked polyvinylpyrrolidone (crospovidone), and low-substituted hydroxypropylcellulose, particularly preferred, the disintegrants are selected from the group consisting of sodium starch glycolate and croscarmellose sodium salt;
   the lubricants are selected from the group consisting of stearic acid, talc, sodium stearyl fumarate and magnesium stearate, particularly preferred, the lubricant is magnesium stearate;
   the binding agents are selected from the group consisting of polyvinyl pyrrolidone (Povidone), copolymers of vinylpyrrolidone with other vinylderivatives (Copovidone), hydroxypropyl methylcellulose, methylcellulose, hydroxypropylcellulose, powdered acacia, gelatin, guar gum, carbomer such as carbopol, polymethacrylates and starch, particularly preferred, the binding agents are selected from the group consisting of hydroxypropyl methylcellulose and copovidone;
   the diluents are selected from carbohydrates such as monosaccharides like glucose, oligosaccharides like sucrose, anhydrous lactose and lactose monohydrate, and sugar alcohols like sorbitol, mannitol, erythrol, and xylitol, particularly preferred the diluent is sorbitol;
   the glidants are selected from the group consisting of colloidal silica, hydrophobic colloidal silica and magnesium trisilicate, such as talcum, particularly preferred the glidants are selected from the group consisting of colloidal silica and hydrophobic colloidal silica; and/or
   the sweeteners are selected from the group consisting of aspartame, saccharin sodium, dipotassium glycyrrhizinate, aspartame, stevia, thaumatin, and the like.
(9) The process according to any of the previous items, wherein to the first and to the second composition the same excipients are added.
(10) The process according to any of the previous items, wherein to the first and to the second composition microcrystalline cellulose, lactose, croscarmellose sodium, hydroxypropyl methylcellulose, colloidal silica and magnesium stearate are added.
(11) The process according to any of the previous items, wherein the dosage form is a tablet and wherein no coating is applied onto the tablets of step (c).
(12) A process for preparing dosage forms containing simvastatin and ezetimibe,
   wherein the process involves the use of direct compression.
(13) The process according to the previous item, wherein the process is further carried out according to any of items (1) to (11).
(14) A dosage form, obtained according to a process according to any of the previous items.
(15) The dosage form according to any of the previous two items, wherein the dosage form is a tablet, preferably a bilayered tablet.
(16) A dosage form, comprising at least two separate compartments, wherein one compartment contains simvastatin and another compartment contains ezetimibe.
(17) The dosage form according to the previous item, wherein each of the compartments respectively constitutes a layer.
(18) The dosage form according to any of the previous two items, wherein the dosage form is a tablet, preferably a bilayered tablet, which consists of two separate compartments, each of which constitutes a layer.
(19) The dosage form according to any of the previous three items, wherein no antioxidants and/or oxygen absorbers are contained.
(20) The dosage form according to any of the previous four items, wherein the dosage form does not comprise a coating layer.
(21) The dosage form according to any of the previous four items, wherein the at least two layers additionally contain one or more excipients.
(22) The dosage form according to the previous item, wherein the excipients are selected from the group consisting of diluents, binding agents, fillers, disintegrants, lubricants, sweeteners, glidants, flavourings and colouring agents, wherein the the dosage form preferably includes excipients selected from those specified in item (8).
(23) The dosage form according to any one of the previous six items, wherein the two layers contain microcrystalline cellulose, lactose, croscarmellose sodium, hydroxypropyl methylcellulose, colloidal silica and magnesium stearate.
(24) The dosage form according to any of the previous three items, wherein the one and the other compartments contain the same type of excipients.
(25) The dosage form according to any one of the previous nine items, wherein the dosage form is a tablet composed of two layers.

### Detailed description of the invention

The present invention is now described in more detail by preferred embodiments and examples, which are however presented for illustrative purpose only and shall not be understood as limiting the scope of the present invention in any way.

It was surprisingly found that a process according to the present invention can provide dosage forms with multiple compartments, notably bilayered tablets, that are more stable than known one-compartment dosage forms, notably monolayered tablets. Increased stability was unexpected, since the two combined, active substances simvastatin and ezetimibe did not exhibit incompatibilities on preformulation stability testing. Without wishing to be bound by any theory, it is presently assumed that the reason for incompatibilities of ezetimibe and simvastatin in known dosage forms is the amplification of undesired interactions between active compounds during co-processing and especially during compression, and eventually in the final dosage form.

Advantageously, the process according to the present invention allows providing stable dosage forms containing simvastatin and ezetimibe without the addition of antioxidants being necessary. In addition, the process according to the invention does not have to be carried out under inert atmosphere to provide stable products but can be carried out while air is present, and the process is therefore easier to carry out and cheaper. Further, the process according to the present invention preferably involves a direct compression step. This allows to avoid the use of solvents (e.g. water) allowing for formulation of solvent sensitive drugs. Furthermore, direct compression is the fastest and most cost effective way of tablet preparation.

The present invention relates to a process for preparing dosage forms comprising the steps of providing a first and a second composition (steps a) and b) of the process). The compositions can be prepared according to known methods by mixing the ingredients of the composition or any other method for the preparations of mixtures suitable for tablet compression (e.g. wet granulation, dry granulation, extrusion /spheronization).

A dosage form according to the present invention is preferably in solid form, including tablets, capsules (soft or hard capsules), caplets, lozenges, and sachets. A dosage form according to the present invention is preferably in the form of a tablet.

The first composition, or first compartment, preferably contains simvastatin in doses of 10 mg, 20 mg, 30 mg, 40 mg, 50 mg, 60 mg, 70 mg, 80 mg, 90 mg or 100 mg, preferably of 60 mg, 70 mg, 80 mg or 90 mg, more preferably of 70 mg or 80 mg, most preferably of 80 mg.

The second composition, or second compartment, preferably contains ezetimibe in doses of 1 mg, 2 mg, 5 mg, 7 mg, 10 mg, 12 mg, 15 mg or 20 mg, preferably of 5 mg, 7 mg, 10 mg or 12 mg, more preferably of 7 mg or 10 mg, most preferably of 10 mg.

To both compositions, additionally one or more pharmaceutically acceptable excipients can be respectively added, wherein, preferably, the addition of antioxidants is omitted. In general, antioxidants are molecules that are capable of slowing down or preventing the oxidation of other molecules. Commonly used antioxidants for the stabilization of dosage forms containing simvastatin and ezetimibe are butylated hydroxyanisole (BHA), ascorbic acid, ascorbyl palmitate, butylated hydroxytoluene (BHT) and sodium metabisulfite. However, the use of antioxidants involves critical shortcomings, as antioxidants can be absorbed through the skin, can be stored in body tissues and are proven to be harmful in higher concentrations. For instance, BHT or BHA cause eye and skin irritation and are irritating to mucous membranes and to the upper respiratory tract. These effects can vary from mild irritation to severe destruction of tissue. Another disadvantage resulting from the use of antioxidants is that such protective compounds may result in the formation of degradation products, which may in turn react with the active substance they were added to preserve.
It is additionally preferred that the compositions as described herein do not contain oxygen absorbers. In general, such oxygen absorbers may be selected from the group of commercially available absorbers such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. The examples of commercially available absorbers are Ageless(TM) (Mitsubishi Gas Chemical), ATCO (Standa Industry), FreshPax(TM) (Multisorb Technologies), O-Buster(TM) (Hsiao Sung Non-Oxygen Chemical Co), Biotika Oxygen Absorber (Biotika) and the like. The same shortcomings and disadvantages as noted above with respect to antioxidants may be associated with the use of oxygen absorbers, and thus their avoidance are likewise beneficial.

However, by using the process according to the present invention, the addition of antioxidants and/or oxygen absorbers is not necessary. Suitable pharmaceutically acceptable excipients, that can be added, include but are not limited to diluents, binding agents, fillers, disintegrants, lubricants, sweeteners, glidants, flavourings and colouring agents.

According to the present invention, any fillers can be used. Preferred fillers are selected from the group consisting of different grades of starches, such as maize starch, potato starch, rice starch, wheat starch, pregelatinized starch, fully pregelatinized starch, cellulose, such as microcrystalline cellulose or silicified microcrystalline cellulose, mannitol, erythritol, lactose, such as lactose monohydrate and lactose anhydrous, calcium, such as calcium hydrogenphosphate, sorbitol, and xylitol, particularly preferred fillers are selected from the group consisting of pregelatinized starch, microcrystalline cellulose, silicified microcrystalline cellulose, lactose monohydrate, and lactose.

According to the present invention, any disintegrants can be used. Preferred disintegrants are selected from the group consisting of carmellose calcium, carboxymethylstarch sodium, croscarmellose sodium salt (cellulose carboxymethylether sodium salt, crosslinked), starch, such as sodium starch glycolate or corn starch, crosslinked polyvinylpyrrolidone (crospovidone), and low-substituted hydroxypropylcellulose, particularly preferred disintegrants are selected from the group consisting of sodium starch glycolate and croscarmellose sodium salt.

According to the present invention, any lubricants can be used. Preferred lubricants are selected from the group consisting of stearic acid, talc, sodium stearyl fumarate and magnesium stearate, a particularly preferred lubricant is magnesium stearate.

According to the present invention, any binding agents can be used. Preferred binding agents are selected from the group consisting of polyvinyl pyrrolidone (povidone), copolymers of vinylpyrrolidone with other vinyl derivatives (copovidone), hydroxypropyl methylcellulose, methylcellulose, hydroxypropylcellulose, powdered acacia, gelatin, guar gum, carbomer such as carbopol, polymethacrylates and starch, particularly preferred binding agents are selected from the group consisting of hydroxypropyl methylcellulose and copovidone.

According to the present invention, any diluents can be used. Preferred diluents are selected from carbohydrates such as monosaccharides like glucose, oligosaccharides like sucrose, anhydrous lactose and lactose monohydrate, and sugar alcohols like sorbitol, mannitol, erythrol, and xylitol, a particularly preferred diluent is sorbitol.

According to the present invention, any glidants can be used. Preferred glidants are selected from the group consisting of colloidal silica, hydrophobic colloidal silica and magnesium trisilicat, such as talcum, particularly preferred glidants are selected from the group consisting of colloidal silica and hydrophobic colloidal silica.

According to the present invention, any sweeteners can be used. Preferred sweeteners are selected from the group consisting of aspartame, saccharin sodium, dipotassium glycyrrhizinate, aspartame, stevia, thaumatin, and the like.

According to the present invention, any flavourings and colouring agents that are known to a person skilled in the art can be used.

The aforementioned excipients can be used for the first and the second composition, wherein, according to the invention, the two compositions may preferably contain the same excipients; however, the excipients can also be independently selected for each composition. Additionally preferred, the first and the second composition contain microcrystalline cellulose, lactose, croscarmellose sodium, hydroxypropyl methylcellulose, colloidal silica and magnesium stearate.

In addition to steps a) and b), the process according to the invention comprises process step c). In process step c) at least two, preferably two, separate compartments are formed using the first and the second composition. In other words, one compartment can be formed using either a composition containing simvastatin or containing ezetimibe (first composition), and the other compartment can be formed using the respective other active ingredient (second composition). Further (third, fourth, etc.) compositions and thus corresponding compartments may be freely chosen, too.

The term "compartment" within the meaning of the present invention denotes a part of the dosage form consisting of the pharmaceutically active ingredient and optionally excipients, preferably as homogenous mixture of components, wherein different compartments consist of different mixtures which differ at least in the type of active ingredient. According to the present invention, the active ingredients are simvastatin and ezetimibe. In each compartment, only one type of active ingredient among simvastatin and ezetimibe is contained. Additionally preferred, the compartments of the dosage form are in direct contact and not separated by further excipients between the compartments. Also preferred, each compartment contains the total amount of the respective active ingredient to be incuded in the whole pharmaceutical dosage form. This means that the compartment which is formed using the first composition contains the total amount of simvastatin and the compartment which is formed using the second composition contains the total amount of ezetimibe.

Preferably each of the at least two separate compartments, preferably two, respectively constitutes a layer. Particularly preferred, according to the present invention, the dosage form is a tablet that consists of two separate compartments, each of which constitutes a layer.

Additionally preferred, no coating is applied onto the tablets of step (c). By carrying out the process according to the invention, as described above, it is not necessary to take measurements that reduce the contact between the dosage form and the oxygen containing environment. These measurements are for instance the application of film coatings, which prevent environmental gases to ingress into the cores. In an additionally preferred embodiment of the invention, the process is carried out in the presence of air. This means that it is not necessary to carry out the process in inert atmosphere, as described above.

The formulations of the present invention may be prepared by well known technological processes, preferably, the process involves a direct compression step, dry granulation and/or lyophilization. Preferably, the layers of the dosage form are compressed onto each other, but any other means, such as core coating, powder layering or core / mantle tablet compression can be employed. According to a particularly preferred process of the present invention, the second layer is compressed onto the first layer. In a further preferred embodiment of the present invention, the process involves a direct compression step. Direct compression, relative to other process technologies, has the advantage that it eliminates the need for solvents such as water and therefore allows the formulation of solvent sensitive drugs. Furthermore, direct compression is the fastest and most cost effective way of tablet preparation. In order to carry out direct compression, preferably a tablet press capable of compressing multilayered, particular bilayered, tablets is used. Such a tablet press can be any tablet press that fulfils the above criteria, preferably a Riva bilayered tablet press is used. For preparing a tablet according to the present invention, the first and second compositions may be compressed in a bilayered tablet press in a well known bilayer tabletting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN. During bilayered tablet compression adequate bond formation between the two layers is achieved by virtue of distance attraction forces (intermolecular forces) and mechanical interlocking between the particles.

The present invention also relates to a dosage form, preferably a tablet, obtained according to a process of the present invention.

The present invention further relates to dosage forms comprising at least two, preferably two, separate compartments containing simvastatin and ezetimibe, respectively. The respective compartments and compositions for forming the compartments as well as methods for preparing such dosage forms have been described above.

In a preferred embodiment, no antioxidants and/or no oxygen absorbers are contained in the dosage form as described above, and particularly preferred, the dosage form does not comprise a coating layer. Further preferred, the dosage form contains additionally one or more excipients, as it is described above.

The following examples illustrate the process of the present invention and are not intended to limit the scope of the invention set forth in the claims appended hereto.

### Examples

### REFERENCE EXAMPLE:

Preformulation compatibility / stability studies of ezetimibe and simvastatin mixtures

| | | |
|---|---|---|
| Sample | Storage conditions: 40/75 - 40±2 ºC / 75±5 % RH (climatic chamber) | Sum of impurities (%) |
| Simvastatin : Ezetimibe = 1:1 | initial | 0.44 |
| Binary mixture | 40/75, 1 month (packed in glass vials closed with stoppers) | 0.42 |
| | 40/75 (open dish), 15 days (packed as open dish) | 0.40 |
| | | |
| Simvastatin : Ezetimibe = 1:8 | initial | 0.38 |
| Binary mixture | 40/75, 1 month (packed in glass vials closed with stoppers) | 0.36 |
| | 40/75 (open dish), 15 days (packed as open dish) | 0.36 |

### COMPARATIVE EXAMPLE 1: Monolayered tablets

For comparison a dosage form was produced where both active ingredients, simvastatin and ezetimibe, are present in monolayered tablets, i.e. within one common component.

### Tablet composition:

| Substance | Amount per tablet (mg) |
|---|---|
| Ezetimibe | 10.00 |
| Simvastatin | 80.00 |
| Silicified microcrystalline cellulose | 120.00 |
| Lactose | 518.00 |
| Croscarmellose sodium | 24.00 |
| Hydroxypropyl methylcellulose | 32.00 |
| Magnesium stearate | 8.00 |
| Colloidal silica | 8.00 |
| Total mass of the tablet (mg) | 800.00 |

### Manufacturing procedure:

Ezetimibe, simvastatin, silicified microcrystalline cellulose, lactose, croscarmellose sodium, hydroxypropyl methylcellulose and colloidal silica were homogeneously mixed. Magnesium stearate was added, the mixture was mixed again and compressed into monolayered tablets.

### Stability characteristics of the tablets:

| Sample | Storage conditions: 40/75 - 40±2 ºC / 75±5 % RH (climatic chamber) | Degradation product 1 | Sum of impurities |
|---|---|---|---|
| Tablets - Comparative Example 1 | initial | 0.13 (%) | 0.81 (%) |
| | 40/75, 1 month (packed in glass vials closed with stoppers) | 0.50 (%) | 1.06 (%) |

### EXAMPLE 1: Bilayered tablets.

According to an example, a dosage form was produced where both active ingredients, simvastatin and ezetimibe, are respective present in different layers of bilayer tablets as an example of different components.

### Tablet composition:

| Substance | Amount per tablet (mg) |
|---|---|
| Ezetimibe | 10.00 |
| Simvastatin | 80.00 |
| Silicified microcrystalline cellulose | 120.00 |
| Lactose | 518.00 |
| Croscarmellose sodium | 24.00 |
| Hydroxypropyl methylcellulose | 32.00 |
| Magnesium stearate | 8.00 |
| Colloidal silica | 8.00 |
| Total mass of the tablet (mg) | 800.00 |

The above composition is divided into two layers respectively differing in the type of active ingredient. The composition of each layer is as follows:

### Composition of the simvastatin layer:

| Substance | Amount per tablet (mg) |
|---|---|
| Simvastatin | 80.00 |
| Silicified microcrystalline cellulose | 90.00 |
| Lactose | 388.50 |
| Croscarmellose sodium | 18.00 |
| Hydroxypropyl methylcellulose | 24.00 |
| Magnesium stearate | 6.00 |
| Colloidal silica | 6.00 |
| Total mass of the layer (mg) | 612.50 |

### Composition of the ezetimibe layer:

| Substance | Amount per tablet (mg) |
|---|---|
| Ezetimibe | 10.00 |
| Silicified microcrystalline cellulose | 30.00 |
| Lactose | 129.50 |
| Croscarmellose sodium | 6.00 |
| Hydroxypropyl methylcellulose | 8.00 |
| Magnesium stearate | 2.00 |
| Colloidal silica | 2.00 |
| Total mass of the layer (mg) | 187.50 |

### Manufacturing process:

Simvastatin, silicified microcrystalline cellulose, lactose, croscarmellose sodium, hydroxypropyl methylcellulose and colloidal silica were homogeneously mixed.
Magnesium stearate was added, the mixture was mixed again and filled into the first hopper of the bilayered tablet press.
Ezetimibe, silicified microcrystalline cellulose, lactose, croscarmellose sodium, hydroxypropyl methylcellulose and colloidal silica were homogeneously mixed.
Magnesium stearate was added, the mixture was mixed again and filled into the second hopper of the bilayered tablet press.

Bilayered tablets were prepared by compressing first the simvastatin containing mixture to a first layer of the bilayered tablet, followed by the ezetimibe mixture being compressed onto the first layer resulting in a bilayered tablet.

### Stability characteristics of the tablets:

| Sample | Storage conditions: 40/75 - 40±2 ºC / 75±5 % RH (climatic chamber) | Degradation product 1 | Sum of impurities |
|---|---|---|---|
| Bilayered tablets - Example 1 | initial | 0.13 (%) | 0.89 (%) |
| | 40/75, 1 month (packed in glass vials closed with stoppers) | 0.37 (%) | 0.99 (%) |

### COMPARATIVE EXAMPLE 2: Monolayered tablets.

### Tablet composition:

| Substance | Amount per tablet (mg) |
|---|---|
| Ezetimibe | 10.00 |
| Simvastatin | 80.00 |
| Microcrystalline cellulose | 120.00 |
| Lactose | 518.00 |
| Croscarmellose sodium | 24.00 |
| Hydroxypropyl methylcellulose | 32.00 |
| Magnesium stearate | 8.00 |
| Colloidal silica, hydrophobic | 8.00 |
| Total mass of the tablet (mg) | 800.00 |

### Manufacturing process:

Ezetimibe, simvastatin, microcrystalline cellulose, lactose, croscarmellose sodium, hydroxypropyl methylcellulose and hydrophobic colloidal silica were homogeneously mixed. Magnesium stearate was added, the mixture was mixed again and pressed into monolayered tablets.

### Stability characteristics of the tablets:

| Sample | Storage conditions: 40/75 - 40±2 ºC / 75±5 % RH (climatic chamber) | Degradation product 1 | Sum of impurities |
|---|---|---|---|
| Tablets - Comparative Example 2 | initial | 0.10 (%) | 0.43 (%) |
| | 40/75, 1 month (packed in glass vials closed with stoppers) | 0.50 (%) | 0.77 (%) |

### EXAMPLE 2: Bilayered tablets.

### Tablet composition:

| Substance | Amount per tablet (mg) |
|---|---|
| Ezetimibe | 10.00 |
| Simvastatin | 80.00 |
| Microcrystalline cellulose | 120.00 |
| Lactose | 518.00 |
| Croscarmellose sodium | 24.00 |
| Hydroxypropyl methylcellulose | 32.00 |
| Magnesium stearate | 8.00 |
| Colloidal silica, hydrophobic | 8.00 |
| Total mass of the tablet (mg) | 800.00 |

The above composition is divided into two layers, the compositions of each layer is as follows.

### Composition of the simvastatin layer:

| Substance name | Amount per tablet (mg) |
|---|---|
| Simvastatin | 80.00 |
| Microcrystalline cellulose | 90.00 |
| Lactose | 388.50 |
| Croscarmellose sodium | 18.00 |
| Hydroxypropyl methylcellulose | 24.00 |
| Magnesium stearate | 6.00 |
| Colloidal silica, hydrophobic | 6.00 |
| Total mass of the layer (mg) | 612.50 |

### Composition of the ezetimibe layer:

| Substance name | Amount per tablet (mg) |
|---|---|
| Ezetimibe | 10.00 |
| Microcrystalline cellulose | 30.00 |
| Lactose | 129.50 |
| Croscarmellose sodium | 6.00 |
| Hydroxypropyl methylcellulose | 8.00 |
| Magnesium stearate | 2.00 |
| Colloidal silica, hydrophobic | 2.00 |
| Total mass of the layer (mg) | 187.50 |

### Manufacturing process:

Simvastatin, microcrystalline cellulose, lactose, croscarmellose sodium, hydroxypropyl methylcellulose and hydrophobic colloidal silica were homogeneously mixed. Magnesium stearate was added, the mixture was mixed again and filled into the first hopper of the bilayered tablet press.
Ezetimibe, microcrystalline cellulose, lactose, croscarmellose sodium, hydroxypropyl methylcellulose and hydrophobic colloidal silica were homogeneously mixed. Magnesium stearate was added, the mixture was mixed again and filled into the second hopper of the bilayered tablet press.
Bilayered tablets were prepared by compressing first the simvastatin containing mixture to a first layer of the bilayered tablet, followed by the ezetimibe mixture being compressed onto the first layer resulting in a bilayered tablet.

### Stability characteristics of the tablets:

| Sample | Storage conditions: 40/75 - 40±2 ºC / 75±5 % RH (climatic chamber) | Degradation product 1 | Sum of impurities |
|---|---|---|---|
| Bilayered tablets - Example 2 | initial | 0.10 % | 0.35 (%) |
| | 40/75, 1 month (packed in glass vials closed with stoppers) | 0.30% | 0.59 (%) |

## Claims

1. A process for preparing dosage forms comprising simvastatin and ezetimibe, said process comprising the steps of:
a) providing a first composition containing simvastatin,
b) providing a second composition containing ezetimibe, and
c) forming a dosage form comprising at least two separate compartments,
wherein one compartment is formed using either the first or the second composition and another compartment is formed using the respective other composition.

2. The process according to claim 1, wherein each of the compartments respectively constitutes a layer.

3. The process according to claims 1 or 2, wherein the dosage form is a tablet that consists of two separate compartments, each of which constitutes a layer.

4. The process according to any of claims 1 to 3, wherein steps (a) to (c) are carried out in the presence of air.

5. The process according to any of claims 1 to 4, wherein the first composition forms the first layer and the second composition forms the second layer, wherein the second layer is compressed onto the first layer.

6. The process according to any one of claims 1 to 5, wherein to the first and to the second composition one or more excipients are respectively added, and wherein the addition of antioxidants and/or oxygen absorbers is omitted.

7. The process according to any of claims 1 to 6, wherein no coating is applied onto the tablets of step (c).

8. A process for preparing dosage forms containing simvastatin and ezetimibe,
wherein the process involves the use of direct compression.

9. A dosage form, obtained according to a process according to any of claims 1 to 8.

10. A dosage form, comprising at least two separate compartments, wherein one compartment contains simvastatin and another compartment contains ezetimibe.

11. The dosage form according to claim 10, wherein each of the compartments respectively constitutes a layer.

12. The dosage form according to claim 10 or 11, wherein the dosage form does not comprise a coating layer.

13. The dosage form according to any of claims 10 to 12, wherein the at least two layers additionally contain one or more excipients.

14. The dosage form according to any of claims 10 to 13, wherein to the one and the other compartments contain the same type of excipients.

15. A combination dosage form comprising a combination of simvastatin and ezetimibe present in two separate compartments of the dosage form, for use in the prophylaxis or treatment of hypercholesterolemia.
